# EUROPÄISCHE PATENTANMELDUNG

(11) **EP 1 270 580 A1**
(43) Veröffentlichungstag der Anmeldung: **02.01.2003**
(21) Anmeldenummer: 02012476.4
(22) Anmeldetag: 12.06.2002
(51) Int. Cl.: C07F 1/00, C07F 3/00, C07D 291/06, C07D 213/20, A61K 7/16, A61K 7/22, A61K 31/54, A61K 31/4425, A61K 33/24, A61P 31/00

(54) **Antimikrobiell wirksame Acesulfam-Komplexe, Verfahren zu ihrer Herstellung und ihre Verwendung**

(30) Priorität: 22.06.2001 DE 10130298
(71) Anmelder: Nutrinova Nutrition Specialties & Food Ingredients GmbH, 65929 Frankfurt am Main (DE)
(72) Erfinder: Burgard, Andreas, Dr., 65929 Frankfurt am Main (DE)
(74) Vertreter: Schweitzer, Klaus, Dr.

(57) **Zusammenfassung**

Komplexe Verbindungen enthaltend Acesulfam besitzen eine gute antimikrobielle Wirkung, insbesondere antibakterielle Wirkung und zeichnen sich dadurch aus, dass sie Komplexverbindungen von metallischen Elementen wie Zink, Kupfer, Silber oder nichtmetallischen quartären Ammoniumkationen, wie dem Cetylpyridiniumkation, mit Acesulfam sind. Die Erfindung betrifft auch ein Verfahren zum Herstellen der Verbindungen und deren Verwendung im Bereich der Mundhygiene.

## Beschreibung

Die vorliegende Erfindung betrifft komplexe Verbindungen enthaltend Acesulfam, die eine antimikrobielle Wirkung, insbesondere antibakterielle Wirkung, besitzen.

Verschiedene Metallkationen wie z.B. Zink(II)-, Kupfer(II)- und Silber(I)-ionen sowie quartäre Ammoniumkationen wie z.B. das Cetylpyridiniumkation wirken antimikrobiell und werden deshalb zur Beseitigung bzw. zur Reduktion von unerwünschten Mikroorganismen wie z.B. Bakterien und Viren in Lebens- und Futtermitteln angewendet. Auch in Mundpflegemitteln und Pharmazeutika werden antimikrobiell wirksame Kationen gegen Bakterien und Viren, die im Mund-Rachen-Raum zu Infektionen wie z.B. Stomatitis, Laryngitis, Herpes oder Karies führen, häufig eingesetzt.

Zink(II)-ionen sind in der WO 97/40812 und der WO 00/00166 als Wirkstoff gegen Mundgeruch, Zahnbelag, Zahnstein und Infektionen im Mundbereich beschrieben. Es ist bekannt, dass Zink(II)-salze die Proteolyse durch direkte Wirkung auf die Proteasen der Bakterien inhibieren. Dabei handelt es sich um Cystein- und Methionin-Proteasen, die durch Freisetzung von Schwefelverbindungen Mundgeruch verursachen können (Marsh, PD, J. Clin. Peridontal 18 (6) 462-467, 1991). Die WO 00/62738 beschreibt einen Lippenstift gegen Mundgeruch, der Zink(II)citrat als antibakteriellen Wirkstoff enthält.

Zink(II)-ionen entfalten bekanntlich ihre antibakterielle Wirkung insbesondere gegen solche Bakterien, die Karies verursachen, wie z.B. streptococcus mutans (siehe Watson, G. K.; Cummins, D.; Van der Ouderaa, F. J. G. Caries Res. (1991), 25(6), 431-7). Colgate PerioGard Plus® Zahn-creme enthält z.B. Zink(II)citrat als antikariösen Wirkstoff.

Die antivirale Wirksamkeit von Zink(II)-ionen gegenüber Herpes Simplex- und Rhinoviren ist in zahlreichen Publikationen und Patenten (z.B. Arens, Max; Travis, Sharon, J. Clin. Microbiol. (2000), 38(5), 1758-1762; WO 99/55342) beschrieben worden.

Kupfer(II)-ionen werden auf Grund ihrer antibakteriellen Wirkung, insbesondere in der Mundhygiene, in Form von Citraten, Bisglycinaten oder Bicarbonaten eingesetzt (siehe US-PS 4,332,791, US-PS 4,652,444, US-PS 5,037,634 und US-PS 5,389,360).

Silber(I)-ionen werden gelegentlich für die Aufbereitung und Entkeimung von Trinkwasser in kleinen Mengen eingesetzt, die ausreichen, um eine Dauerentkeimung und Dauerfrischhaltung des Wassers infolge der bakteriziden Wirkung der Silberverbindungen zu bekommen (siehe WO 96/01231, US-PS 5,366,636).

K. Yamamoto et al. (Dent. Mater. (1996), 12(4), 227-229) berichten über die antibakterielle Aktivität von Silber(I)ionen, die in Silikatfüllungen implantiert sind, gegen im Mund vorkommende Streptokokken.

Silber(I)-ionen werden außerdem zur Raucherentwöhnung in Mundspülungen eingesetzt (siehe US-PS 5,032,612). Beim Rauchen entsteht in Anwesenheit von Silber(I)ionen aus Nikotin Nikotinsäureamid, welches einen unangenehmen Geschmack im Mund verursacht, so dass eine Aversion gegenüber dem Zigarettenrauch entstehen soll (US-PS 5,122,366). Silber(I)acetat, eingearbeitet in Lutschtabletten, soll ebenfalls für erfolgreiche Raucherentwöhnungstherapien geeignet sein (siehe US-PS 4,832,994).

Cetylpyridiniumkationen wirken als quartäre Ammoniumverbindungen antibakteriell und werden insbesondere in Mundwässern eingesetzt (siehe WO 00/51559, WO 00/44338). In der EP-A-0 399 479 und der GB-A 2348370 wird ein Kaugummi zur Mund- und Rachendesinfektion beschrieben, in dem Cetylpyridiniumkationen den antibakteriell wirksamen Bestandteil bilden. In der WO 97/38586 wird über eine Methode berichtet, die eine Inhibierung bzw. Beseitigung von Bakterien in kontaminierten Lebensmitteln mittels quartären Ammoniumverbindungen erreichen soll. Diese Methode verwendet quartäre Ammoniumverbindungen, insbesondere Cetylpyridiniumchlorid, die in Lebensmitteln Mikroorganismen wie z.B. Staphylococcen, Listerien, Salmonellen, Escherichia Coli-Bakterien und Pilze wie Aspergillus flavus beseitigt. Die quartären Ammoniumverbindungen werden in gelöster Form durch Aufsprühen auf Lebensmittel wie Fleisch, Fisch, Gemüse und Früchte appliziert.

Außerdem werden Cetylpyridiniumkationen in zahlreichen pharmazeutischen Präparaten wie z.B. in ®Hextriletten von Warner Lambert, ®Dobendan von Boots Healthcare und ®Frubienzym von Boehringer Ingelheim Pharma KG als Lutschtabletten bei Infektionen des Mund-, Hals- und Rachenraumes, die durch Viren und Bakterien verursacht sind, eingesetzt. Cetylpyridinium-chlorid lagert sich an die Zellwände der Krankheitserreger an, zerstört sie und tötet die Keime ab.

Auch Colgate PerioGard Plus® Mundspülung enthält Cetylpyridiniumchlorid in wirksamen Mengen.

Auch synergistische Kombinationen von Zink(II)-salzen und Cetylpyridiniumchlorid gegen Mundgeruch sind in der WO 00/51559 beschrieben.

Der Schwerpunkt der vorliegenden Erfindung liegt auf dem Gebiet der Anwendung von antibakteriellen wirksamen Kationen insbesondere im Bereich der Mundhygiene. Die Volkskrankheit Karies, die durch Bakterien wie z.B. Streptococcus mutans verursacht wird, kann mit antibakteriell wirksamen Kationen eingedämmt werden. Mundgeruch wird in vielen Fällen durch Bakterien verursacht, die im Mund vorhandene Essensreste zersetzen und dabei schwefelhaltige Verbindungen freisetzen. Ältere Menschen und Diabetespatienten klagen oft über Mundtrockenheit (Xerostomia), die durch eine verminderte Speichelproduktion im Mund bedingt ist. Mundtrockenheit ist ein idealer Nährboden für Bakterien, so dass Infektionen, Mundgeruch und Karies gerade bei Diabetes signifikant zu beobachten sind. Essensreste und Bakterien können bei Xerostomia durch den fehlenden Speichel nicht mehr weggewaschen werden. Die Folge ist, dass sich die Bakterien im Mund auf dem reichlich vorhandenen Nährboden besser vermehren können. Damit pathogene Keime und aus diesen hervorgerufene Folgen wie z.B. Karies oder Mundgeruch reduziert bzw. eliminiert werden können, werden antimikrobiell wirksame Kationen in Zahnpasta, Kaugummis und Mundspülungen eingesetzt.

Problematisch ist allerdings, dass viele Salze der oben beschriebenen antimikrobiell wirksamen Kationen bereits in geringen Mengen unangenehm schmecken und insbesondere einen adstringierenden Geschmack aufweisen. Die Einarbeitung von Verbindungen dieser antibakteriell wirksamen Kationen in Lebensmittel, Mundpflegemittel, Arzneimittel oder Futtermittel und die Zufuhr über spezielle Präparate ist deshalb auf Grund mangelnder Akzeptanz durch Mensch und Tier ein schwieriges Unterfangen.

Aufgabe der vorliegenden Erfindung war somit die Schaffung einer möglichst angenehm schmeckenden Form antimikrobieller, insbesondere antibakterieller, Wirkstoffe, mit denen sich die Akzeptanz und die Möglichkeiten gezielter Anwendung der Wirkstoffe in Lebensmitteln, Mundpflegemitteln, Arznei- und Futtermitteln deutlich verbessern lässt.

Überraschend wurde gefunden, dass der bekannte Süßstoff Acesulfam (6-Methyl-3,4-dihydro-1,2,3-oxathiazin-4-on-2,2-dioxid), der bisher nur als Kaliumsalz (Acesulfam-K) angeboten wird, von dem aber auch Salze mit Alkali- und Erdalkalimetallen, z.B. Natrium-, Kalium-, Magnesium- und Calciumsalze, das sauer reagierende Acesulfam selbst, das die sogenannte Acesulfamsäure darstellt, und einige Aminsalze bekannt sind, mit diesen antimokrobiell, insbesondere antibakteriell, wirksamen Kationen stabile Komplexe mit dem Acesulfam-Anion bildet. Diese Komplexe zeichnen sich überraschend durch einen angenehm süßen Geschmack aus, dem die adstringierende Note vieler Salze der antibakteriell wirksamen Kationen nicht anhaftet.

Die vorliegende Erfindung löst die Aufgabe somit durch Komplexverbindungen von metallischen Elementen wie Zink, Kupfer, Silber oder nichtmetallischen quartären Ammoniumkationen, wie z.B. dem Cetylpyridiniumkation, mit Acesulfam.

Erfindungsgemäß handelt es sich dabei bevorzugt um Komplexe des Acesulfams, die aus einem Metall bzw. einem quartären Ammoniumion und einem oder zwei Acesulfam-Molekülen zusammengesetzt sind und die darüber hinaus gegebenenfalls Kristallwasser enthalten können. Die erfindungsgemäßen Komplexe sind stöchiometrisch definierte Verbindungen, bei denen das Metall bzw. das quartäre Ammoniumion kationisch und die Acesulfammoleküle anionisch vorliegen.

Die erfindungsgemäßen Verbindungen sind stabil und zerfallen auch bei der Einarbeitung in Kaugummi oder anderen antimikrobiell wirksamen Zubereitungen nicht. Sie haben außerdem den Vorteil, dass sie sich als Salze nicht entmischen, was geschmackliche Inhomogenitäten zur Folge hätte. Solche Entmischungen sind ein in der Lebensmittel- und Arzneimittelherstellung bekanntes Problem.

Insbesondere können die erfindungsgemäßen Komplexe in Zwischen- oder Vorprodukten eingesetzt werden, ohne dass sich das antimikrobiell wirksame Kation und das Acesulfam entmischen. Sie lassen sich deshalb problemlos in die Zubereitungen einarbeiten, mit denen sie aufgenommen werden sollen, z. B. Kaugummi, Kautabletten, Komprimate oder andere Zubereitungen zur oralen Applikation.

Dadurch, dass die Metallkationen oder das quartäre Ammoniumkation in diesen Komplexen bevorzugt salzartig gebunden sind, sind sie nach Solvolyse durch Wasser ohne weiteres antimikrobiell wirksam. Mit der vorliegenden Erfindung wird die antimikrobielle Wirksamkeit dieser Acesulfam-Komplexe im Vergleich zu sonst eingesetzten Salzverbindungen wie Zink(II)-sulfat, Kupfer(II)-sulfat, Silber(I)-nitrat und Cetylpyridiniumchlorid durch die antibakterielle Potenz gegenüber Bakterien wie *Streptococcus mutans* (ATCC 25175), *Actinomyces odontolyticus* (ATCC 17982) und *Staphylococcus aureus* (ATCC 6538) durch die Bestimmung der minimalen Hemmkonzentration beispielhaft gezeigt. Die antibakterielle Wirksamkeit gegenüber weiteren Bakterien und die antiviralen Eigenschaften dieser Acesulfam-Komplexe sind durch die Beispiele nicht ausgeschlossen und kann auch auf andere Bakterien und Viren bezogen werden.

### Methode zur Bestimmung der minimalen Hemmkonzentration

Die Proben werden zur Bestimmung der minimalen Hemmkonzentration in WSH (Wasser standartisierter Härte) gelöst und mit doppelt konzentrierter Caso-Bouillon pH 5,5 vermischt. Nach Beimpfen mit den Prüfkeimen wurden die Ansätze der Prüfkeime *Streptococcus mutans* (ATCC 25175) und *Actinomyces odontolyticus* (ATCC 17982) mit Paraffin überschichtet und über einen Zeitraum von drei Tagen bei einer Temperatur von 37 °C bebrütet.

Der Ansatz des Prüfkeimes *Staphylococcus aureus* (ATCC 6538) wurde zwei Tage bei 37°C bebrütet. Anschließend wurden die Proben auf Caso-Agar ausgestrichen und für zwei Tage bei 37°C bebrütet.

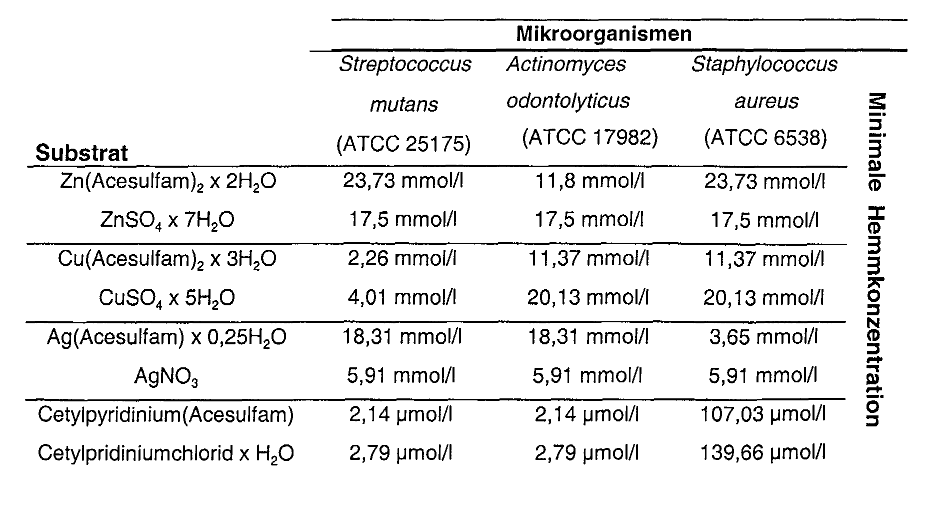

Die Werte der minimalen Hemmkonzentrationen aus der vorstehenden Tabelle zeigen, dass die jeweiligen Acesulfam-Komplexe in der gleichen Größenordnung ihre antibakterielle Aktivität entfalten, wie die jeweiligen einfachen anorganischen Salze. In den meisten Fällen ist die antibakterielle Aktivität der Acesulfam-Komplexe sogar noch besser, d.h. die minimale Hemmkonzentration ist geringer als beim entsprechenden anorganischen Salz wie z.B. im Fall der Cetylpyridiniumverbindungen. Die bessere antibakterielle Aktivität des Cetylpyridinium-Acesulfam im Vergleich zu dem Cetylpyridiniumchlorid kann durch frühere Beobachtungen, dass Süßstoffe wie auch das Acesulfam-K gegenüber Bakterien inhibierend wirken, erklärt werden

In der DE-A 34 37 090 wird die inhibierende Wirkung von Zahnkaries durch Hemmung des Zuckerstoffwechsels von Mundbakterien wie *Streptococcus mutans* mittels Acesulfam-K, bzw. dessen Kombination mit anderen Süßstoffen wie Saccharin und Cyclamat beschrieben. Auch die DE-A 32 11 258 berichtet über ein Verfahren zur Behandlung von Zähnen zur Unterdrückung oder zur Vorbeugung von Karies, bei dem man die Zähne mit Acesulfam-K in einer ausreichenden Menge in Berührung bringt, um das Wachstum von *Streptococcus mutans*-Stämmen in der Mundhöhle oder auf den Zähnen zu inhibieren. Allerdings führen die für eine wirksame Unterdrückung von Karies nötigen Mengen an Acesulfam-K zur völligen Übersüßung, die geschmacklich nicht mehr akzeptabel ist.

M. Pfeffer et al. berichten über eine Hemmung der Glucosevergärung bei *Streptococcus mutans*-Bakterien in Gegenwart der Süßstoffe Acesulfam-K, Cyclamat und Saccharin (siehe Z. Ernährungswiss. 24, 1985, 231-235). Die Interpretation der Versuche von M. Pfeffer et al. führt im Rahmen der Zellphysiologie zu dem Vorschlag, dass die Süßstoffe auf Glucose-Transportsysteme in der bakteriellen Zytomembran wirken.

Bei Silbernitrat ist der stärkere antibakterielle Effekt im Vergleich zum Silber-Acesulfam-Komplex wahrscheinlich durch die zusätzlich antibakterielle Eigenschaft des Nitratanions zu erklären.

Die erfindungsgemäßen neuen Acesulfam-Komplexe können in den gängigen Verarbeitungsschritten von Lebensmitteln und in Arzneimitteln und kosmetischen Mitteln z.B. Zahnpflege- und Mundpflegemittel, oder in Futtermitteln z.B. in Form eines sog. Prä-mix problemlos eingesetzt werden. Die Verarbeitung ist einfach und erfolgt nach bekannten Methoden. Bei festen Zubereitungen werden die erfindungsgemäßen Komplexe in fester Form, ggf. in geeigneter Korngröße, mit den anderen Zutaten vermischt. Zur Verwendung in Tabletten, Komprimaten und vergleichbaren Produkten sowie pulverförmigen Zubereitungen können sie mit anderen dafür geeigneten Zutaten granuliert und als Granulat weiterverarbeitet werden. Auf Grund ihrer guten Löslichkeit können sie aber auch leicht in flüssigen Produkten aus den genannten Bereichen eingesetzt oder in Form ihrer wässrigen Lösungen verarbeitet werden.

Schließlich betrifft die vorliegende Erfindung auch ein Verfahren zur Herstellung der erfindungsgemäßen Komplexe. Das als Ausgangssubstanz dienende Acesulfam oder dessen Kaliumsalz, Acesulfam-K, sind im Handel erhältlich oder können, wie auch beliebige andere Acesulfamsalze, nach dem in der EP-A 0 155 634 beschriebenen Verfahren hergestellt werden.

Zur Herstellung der erfindungsgemäßen Komplexe wird ein Verfahren angewandt, mit dem sich andere ionische Bestandteile der für die Herstellung geeigneten Ausgangsstoffe beseitigen lassen. Das kann entweder durch Abtrennung von schwerlöslichen Verbindungen der anderen ionischen Bestandteile oder durch den Einsatz von Ausgangsmaterialien geschehen, mit denen von vornherein nur die antimikrobiell wirksamen Kationen und Acesulfam in einer Lösung verbleiben, aus der die Komplexe in geeigneter Weise isoliert werden. Zu diesen Verfahren gehören
- Die Umsetzung von Salzen des Acesulfams, deren Kationen geeignete schwerlösliche Verbindungen bilden, die sich ausfällen lassen wie insbesondere das Calcium-, aber auch das Bariumsalz des Acesulfams, mit löslichen Salzen der oben beschriebenen Kationen, deren Anionen mit den Kationen des Acesulfamsalzes schwerlösliche Verbindungen bilden, z. B. Sulfate, oder
- die Umsetzung von basischen Carbonaten der antimikrobiell wirksamen Kationen mit Acesulfamsäure (Acesulfam) unter Freisetzung von CO₂.

Jeweils gebildete Niederschläge werden gegebenenfalls abgetrennt, bevor die gewünschten Acesulfam-Komplexe isoliert werden. Diese Isolierung erfolgt bevorzugt durch Kristallisation, z.B. durch Verdampfen des Lösungsmittels, bevorzugt Wasser oder mit Wasser mischbare Lösungsmittel, oder durch Zugabe von mit Wasser mischbaren Lösungsmitteln zum Reaktionsgemisch. Bevorzugte mit Wasser mischbare Lösungsmittel sind z.B. Alkohole.

Die als Ausgangsstoffe der Reaktion dienenden Salze des Acesulfams können z.B. als wässrige Lösung vorgelegt oder aber auch in einer sog. Eintopfreaktion, vor Zugabe des Salzes mit dem antimikrobiell wirkenden Kation, aus Acesulfam und einem geeigneten Erdalkalicarbonat (Ba, Ca-Salz) gebildet werden.

Die Erfindung wird durch die folgenden Ausführungsbeispiele näher erläutert, ohne dadurch in ihrem Umfang beschränkt zu sein:

### Beispiel 1

### Acesulfam-Zink-Komplex:

### Methode 1:

20 mmol (3,947 g) schwerlösliches Bariumcarbonat (oder 20 mmol Calciumcarbonat) wurden in 20 ml Wasser vorgelegt und mit 40 mmol (6,525 g) Acesulfam-H versetzt. Nach Beendigung der CO₂-Entwicklung entstand eine homogene Lösung, aus der mit 20 mmol (0,575 g) Zink(II)sulfat-heptahydrat schwerlösliches Bariumsulfat (oder Calciumsulfat) ausgefällt wurde. Nach Abfiltrieren des Niederschlags und Einengen der Lösung kristallisierte mit 97 % Ausbeute der Acesulfam-Zink-Komplex in Form farbloser Kristalle aus.

### Methode 2:

10 mmol (3,42 g) schwerlösliches basisches Zinkcarbonat-hydrat (ZnCO₃ x 2 Zn(OH)₂ x H₂O) wurden in 20 ml Wasser vorgelegt und mit 60 mmol (9,789 g) Acesulfam-H versetzt. Nach Beendigung der CO₂-Entwicklung entstand eine homogene Lösung. Der Acesulfam-Zink-Komplex kristallisierte mit 99 % Ausbeute nach Einengen der Lösung in Form farbloser Kristalle aus.

### Methode 3:

20 mmol (1,31 g) metallisches Zinkpulver wurden in 20 ml Wasser vorgelegt und mit 40 mmol (6,525 g) Acesulfam-H versetzt. Nach Beendigung der H₂-Entwicklung entstand eine homogene Lösung. Der Acesulfam-Zink-Komplex kristallisierte mit 99 % Ausbeute nach Einengen der Lösung in Form farbloser Kristalle aus.

Der Acesulfam-Zink-Komplex zersetzt sich bei 255°C.

Die Kristallstruktur des Acesulfam-Zink-Komplexes konnte durch eine Röntgenstrukturanalyse aufgeklärt werden.

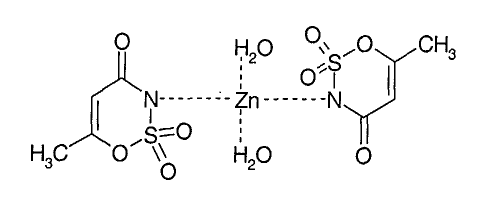

(Ace)₂Zn, 2 C₄H₄NO₄S * Zn * 2 H₂O, Mᵣ = 425.69, monoclinic, C2/c, a = 12.907(4), b = 5.584(2), c = 21.222(8) Å, β = 91.31(3)°, V =1529.2(8) Å³, Z = 4, Dₓ = 1.849 Mg m⁻³, λ (Mo-Kα) = 0.71073 Å, µ = 1.932 mm⁻¹, F(000) = 864, T = 293(2) K, R = 0.0235 and R_{w} = 0.0649 for I > 2σ(I) (1356 reflections), R = 0.0255 and R_{w} = 0.0669 for all 1436 unique CCD data. S(Fₒ² - F_{c}²)² was minimized.

### Beispiel 2

### Acesulfam-Kupfer-Komplex:

### Methode 1:

20 mmol (3,947 g) schwerlösliches Bariumcarbonat (oder 20 mmol Calciumcarbonat) wurden in 20 ml Wasser vorgelegt und mit 40 mmol (6,525 g) Acesulfam-H versetzt. Nach Beendigung der CO₂-Entwicklung entstand eine homogene Lösung, aus der mit 20 mmol (0,499 g) Kupfer(II)sulfat-pentahydrat schwerlösliches Bariumsulfat (oder Calciumsulfat) ausgefällt wurde. Nach Abfiltrieren des Niederschlags und Einengen der Lösung kristallisierte der Acesulfam-Kupfer-Komplex mit 96 % Ausbeute in Form blauer Kristalle aus.

### Methode 2:

10 mmol (2,21 g) schwerlösliches basisches Kupfercarbonat (CuCO₃ x Cu(OH)₂) wurden in 20 ml Wasser vorgelegt und mit 40 mmol (6,526 g) Acesulfam-H versetzt. Nach Beendigung der CO₂-Entwicklung entstand eine homogene blaue Lösung. Der Acesulfam-Kupfer-Komplex kristallisierte mit 98 % Ausbeute nach Einengen der Lösung in Form blauer Kristalle aus.

Der blaugefärbte Acesulfam-Kupfer-Komplex entfärbt sich bei 117°C und zersetzt sich bei 179°C.

Die Kristallstruktur des Acesulfam-Kupfer-Komplexes konnte durch eine Röntgenstrukturanalyse aufgeklärt werden.

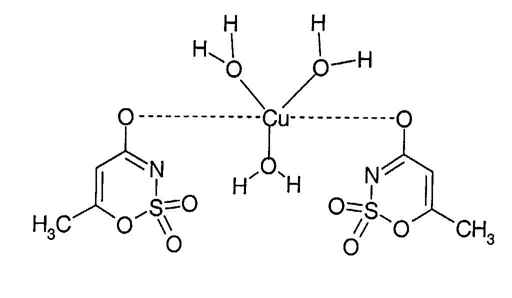

(Ace)₂Cu, 2 C₄H₄NO₄S * Cu * 3 H₂O, Mᵣ = 441.87, monoclinic, C2/c, a = 19.30(2), b = 9.677(9), c = 9.007(8) Å, β = 102.92(2)°, V =1640(3) Å³, Z = 4, Dₓ = 1.790 Mg m⁻³, λ (Mo-Kα) = 0.71073 Å, µ = 1.645 mm⁻¹, F(000) = 900, T = 293(2) K, R = 0.0371 and R_{w} = 0.0753 for I > 2σ(I) (1251 reflections), R = 0.0440 and R_{w} = 0.0791 for all 1448 unique CCD data. S(Fₒ² - F_{c}²)² was minimized.

### Beispiel 3

### Acesulfam-Silber-Komplex:

### Methode 1:

Eine Lösung von 30 mmol (5,148 g) Silber(I)nitrat in 30 ml Wasser wurde einer Lösung von 30 mmol (6,037 g) Acesulfam-K werden in 30 ml Wasser zugegeben. Nach der vollständigen Zugabe wurde der gebildete farblose Niederschlag abgesaugt und zweimal mit je 10 ml Wasser gewaschen. Es resultierten 88 % farblose Acesulfam-Silber-Kristalle. Die Umsetzung und anschließende Aufarbeitung wie oben beschrieben muss nicht unter Lichtausschluss durchgeführt werden.

### Methode 2:

15 mmol (3,48 g) schwerlösliches Silber(I)oxid wurde in 30 ml Wasser vorgelegt und mit einer Lösung von 30 mmol (4,895 g) Acesulfam-H in 30 ml Wasser versetzt. Nach der vollständigen Umsetzung und Aufarbeitung wie in Methode 1 resultierten 80% farblose Acesulfam-Silber-Kristalle.

Die Kristallstruktur des Acesulfam-Silber-Komplexes konnte durch eine Röntgenstrukturanalyse aufgeklärt werden.

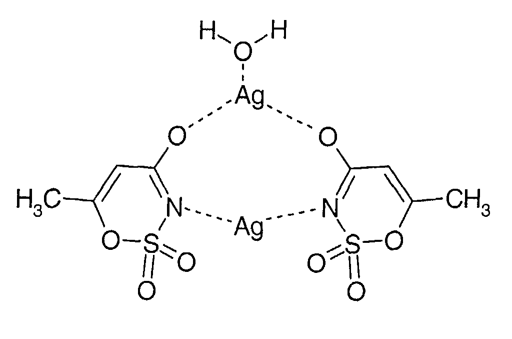

### Crystal data:

crystal system: monoclinic
space group: P2/n ; Z = 8
summation formula:C₄H₈AgNO₄S * 0.25 H₂O
cell dimensions:
   a = 13.438(3) Å
   b = 7.525(2) Å
   c = 15.067(3) Å
   β = 113.111(3)°
cell volume: 1401.3(5) Å³
temperature: 293 (2) K
calculated density: 2.602 Mg/m³
unique reflections: 2584
model quality: R_{obs. data} = 2.15 %

### Beispiel 4

### Acesulfam-Cetylpyridinium-Komplex:

### Methode 1:

10 mmol (3,653 g) Cetylpyridiniumchlorid x H₂O wurden in 25 ml Methanol gelöst und mit 10 mmol (2,012 g) Acesulfam-K suspendiert. Nach 1, 5 Stunden Rühren bei Raumtemperatur wurde der gebildete Niederschlag abfiltriert. Das Filtrat wurde im Vakuum eingeengt. Der ölige Rückstand kristallisierte anschließend aus. Es resultierten 90% farblose, nichthygroskopische Kristalle.

### Methode 2:

10 mmol (3,653 g) Cetylpyridiniumchlorid x H₂O wurden in 75 ml Methylenchlorid suspendiert und mit 10 mmol (2,012 g) Acesulfam-K versetzt. Nach 1, 5 Stunden Rühren bei Raumtemperatur wurde der gebildete Niederschlag abfiltriert. Das Filtrat wurde im Vakuum eingeengt. Der ölige Rückstand kristallisierte anschließend aus. Es resultierten 95% farblose, nichthygroskopische Kristalle.

60-MHz-¹H-NMR (CDCL₃): δ(ppm) = 0.9 (t, 3H, CH₃-Cetylpyridin), 1.25 (m, 28H, CH₂-Cetylpyridin), 2.05 (s, 3H, CH₃-Acesulfam), 4.95 (t, 2H, CH₂-N-Cetylpyridin), 5.6 (s, 1H, CH-Acesulfam), 8.5 (m, 3H, CH-Aromat-Cetylpyridin), 9.6 (d, 2H CH-Aromat-Cetylpyridin).

## Patentansprüche

1. Komplexe Verbindungen enthaltend Acesulfam, die eine antimikrobielle Wirkung, insbesondere antibakterielle Wirkung, besitzen, **dadurch gekennzeichnet, dass** sie Komplexverbindungen von metallischen Elementen wie Zink, Kupfer, Silber oder nichtmetallischen quartären Ammoniumkationen, wie dem Cetylpyridiniumkation, mit Acesulfam sind.

2. Komplexe Verbindungen nach Anspruch 1, **dadurch gekennzeichnet, dass** sie Komplexe des Acesulfams sind, die aus einem Metall oder einem quartären Ammoniumion und einem oder zwei Acesulfam-Molekülen zusammengesetzt sind und die gegebenenfalls zusätzlich Kristallwasser enthalten.

3. Komplexe Verbindungen nach Anspruch 1 oder 2, **dadurch gekennzeichnet, dass** sie stöchiometrisch definierte Verbindungen sind, bei denen das Metall oder das quartäre Ammoniumion kationisch und die Acesulfammoleküle anionisch vorliegen.

4. Verfahren zum Herstellen komplexer Verbindungen nach einem oder mehreren der Ansprüche 1 bis 3, bei dem die Einzelbestandteile der komplexen Verbindungen so in einem Lösungsmittel umgesetzt werden, dass sich andere ionische Bestandteile der für die Herstellung geeigneten Ausgangsstoffe durch Abtrennung von schwerlöslichen Verbindungen der anderen ionischen Bestandteile beseitigen lassen, oder bei dem nur solche Ausgangsmaterialien eingesetzt werden, bei denen nach Beendigung der Reaktion nur die antimikrobiell wirksamen Kationen und Acesulfam in der Lösung verbleiben, wobei die komplexen Verbindungen dann in geeigneter Weise isoliert werden.

5. Verfahren nach Anspruch 4, **dadurch gekennzeichnet dass** Salze des Acesulfams, insbesondere Barium- oder Calziumsalze, umgesetzt werden mit Salzen, deren Anion mit den Kationen des Acesulfamsalzes schwerlösliche Verbindungen bilden, insbesondere mit Sulfaten.

6. Verfahren nach Anspruch 4, **dadurch gekennzeichnet, dass** die Umsetzung von basischen Carbonaten antimikrobiell wirksamer Kationen mit Acesulfamsäure unter Freisetzung von CO₂ vorgenommen wird.

7. Verfahren nach Anspruch 5, **dadurch gekennzeichnet, dass** gebildete Niederschläge abgetrennt werden, bevor die Acesulfam-Komplexe isoliert werden.

8. Verfahren nach einem der Ansprüche 4 bis 7, **dadurch gekennzeichnet, dass** die Isolierung der komplexen Verbindungen durch Kristallisation vorgenommen wird.

9. Verfahren nach einem der Ansprüche 4 bis 8, **dadurch gekennzeichnet, dass** als Lösungsmittel Wasser oder mit Wasser mischbare organische Lösungsmittel eingesetzt werden.

10. Verwendung komplexer Verbindungen nach einem der Ansprüche 1 bis 3 im Bereich der Mundhygiene.
